# EUROPÄISCHE PATENTSCHRIFT

(11) **EP 2 904 978 B1**
(45) Veröffentlichungstag und Bekanntmachung des Hinweises auf die Patenterteilung: **27.07.2016**
(21) Anmeldenummer: 15153600.0
(22) Anmeldetag: 03.02.2015
(51) Int. Cl.: A61B 17/02

(54) **Retraktor und Bedienverfahren**
Retractor and operating method
Rétracteur et procédé de commande

(30) Priorität: 10.02.2014 DE 102014101602
(43) Veröffentlichungstag der Anmeldung: 12.08.2015
(73) Patentinhaber: Karl Storz GmbH & Co. KG, 78532 Tuttlingen (DE)
(72) Erfinder: Merz, Robin, 78532 Tuttlingen (DE); Stefan, Jochen, 78532 Tuttlingen (DE)

(56) Entgegenhaltungen:
- EP-A1- 0 705 566
- WO-A2-00/67642
- DE-A1- 19 840 880

## Beschreibung

Die nachfolgende Erfindung bezieht sich auf einen Retraktor und auf ein Bedienverfahren für den Retraktor.

In der endoskopischen Chirurgie werden Retraktoren verwendet, um Organe aus dem Operationsgebiet zu halten, um freie Sicht und hinreichende Bewegungsfreiheit zu haben. Die Retraktoren haben üblicherweise einen Betätigungsgriff, der über einen Schaft an eine flexible Retraktionsstruktur gekoppelt ist, die aus mehreren Gliedern besteht, die untereinander gelenkig verbunden sind. Zum Einführen in den Körper bzw. zum Durchführen durch einen Trokar sind die Glieder fluchtend mit dem Schaft ausgerichtet und werden erst im Körperinneren zu einen Ring oder Haken zur Aufnahme des Organs geformt. Um zu einem Ring geschlossen zu werden, hat die Retraktionsstruktur am letzten distalen Glied ein Verbindungsmittel, das mit dem Schaft verbunden wird. Der Ring wird mit Hilfe eines zweiten endoskopischen Instruments, etwa einer Fasszange, das durch eine weitere Körperöffnung eingebracht ist, oder mittels eines Bowdenzugs, der in den Gliedern selbst geführt ist und der mit dem Betätigungsgriff betätigt wird, geformt. Die Gelenke, die die einzelnen Glieder verbinden, sind dabei möglichst atraumatisch gestaltet und können Führungsflächen und/oder Winkelanschläge aufweisen, die die einzelnen Gelenke jeweils in einen zum Ringschluss optimalen Winkel bringen.

Ein gattungsgemäßer Retraktor ist aus der DE 199 20 869 A1 bekannt und weist einen Schaft auf, an dessen distalem Ende mehrere gelenkig verbundene Glieder angeordnet sind, die zu einem Ring verstellt werden können, wobei ein distales Glied über zwei Rasthaken an den Schaft angekoppelt werden kann. Die Glieder können mit Hilfe eines Bowdenzugs in die Ringstellung gebracht werden, der innerhalb der Glieder geführt ist. Die Retraktionsstruktur kann gegenüber dem Schaft durch ein Gelenk bis zu 90° abgewinkelt werden.

Neben den dort offenbarten Rasthaken ist auch bekannt, an dem freien Ende der Retraktionsstruktur einen Rastpin anzuordnen, der lösbar in Eingriff mit einem Federdruckstück im Schaft gebracht werden kann. Hierbei ist sowohl beim Koppeln als auch beim Entkoppeln ein großer Kraftaufwand nötig, da stets die Rückstellkraft des Federdruckstücks überwunden werden muss; dies stellt ein großes Verletzungsrisiko dar, da ein Bediener bei der Kraftanwendung stets abrutschen kann. Ferner ist das Federdruckstück nur unter hohem Aufwand zu reinigen bzw. zu desinfizieren. Ausgehend von diesem Stand der Technik liegt der vorliegenden Erfindung die Aufgabe zu Grunde, einen verbesserten Retraktor für die endoskopische Chirurgie zu schaffen, der sicherer und mit weniger Kraftaufwand bedient werden kann, der einfacher zu reinigen ist und der eine hohe Sicherheit in Bezug auf Bedienungsfehler hat.

Diese Aufgabe wird durch einen Retraktor für die endoskopische Chirurgie mit den Merkmalen des unabhängigen Anspruchs 1 gelöst.

Des Weiteren ergibt sich die Aufgabe, ein Bedienverfahren für einen solchen Retraktor zu schaffen, das es ermöglicht, den Retraktor effizienter und zeitsparender einzusetzen.

Diese Aufgabe wird durch ein Bedienverfahren für einen Retraktor mit den Merkmalen des Anspruchs 14 gelöst.

Bevorzugte Ausführungsbeispiele der Vorrichtung und des Verfahrens werden durch die Unteransprüche beschrieben.

Der erfindungsgemäße Retraktor für die endoskopische Chirurgie weist in einer ersten Ausführungsform einen ersten Schaftabschnitt auf, innerhalb dessen eine Betätigungsvorrichtung verschiebbar geführt ist und die schwenkbar mit einem zweiten Schaftabschnitt gekoppelt ist. Ferner weist der Retraktor einen Übertragungsarm auf, der an einem Ende an ein distales Ende der Betätigungsvorrichtung angelenkt ist und an seinem weiteren Ende exzentrisch in Bezug zu einer Drehachse der Schaftabschnitte an den zweiten Schaftabschnitt angelenkt ist. Mit dem distalen Ende des zweiten Schaftabschnitts ist ein Ende einer Retraktionsstruktur verbunden, die anderenends ein Kopplungsende hat. Mit ihrem Kopplungsende kann die Retraktionsstruktur mit einer Kopplungsvorrichtung des zweiten Schaftabschnitts lösbar gekoppelt werden.

Die Kopplungsvorrichtung weist einen Schieber auf, der innerhalb des zweiten Schaftabschnitts verschiebbar geführt ist. Der Übertragungsarm ist über eine Zapfen-Langloch-Verbindung an den zweiten Schaftabschnitt angelenkt, wobei sich ein Langloch der Zapfen-Langloch-Verbindung bei gestreckten Schaftabschnitten parallel zur Längsachse der Schaftabschnitte erstreckt. Der Übertragungsarm ist mit dem Schieber zur Erreichung einer Längsverfahrung desselben operativ gekoppelt, wobei die Kopplungsvorrichtung über die Längsverfahrung des Schiebers in einen Freigabezustand überführt werden kann.

Der Schieber dient als Teil eines Schlosses, das für die Montage geöffnet ist. Hier kann die Spitze bzw. der distale oder auch "freie" Teil des Gliederrings durchgeführt werden. Wird der Schieber dann axial bewegt, wird ein Hinterschnitt erzeugt, der die Spitze des Gliederrings fixiert und gegen Herausziehen sperrt.

"Innerhalb" soll hierin bedeuten, dass die Betätigungsvorrichtung in dem ersten Schaftabschnitt und der Schieber in dem zweiten Schaftabschnitt entlang der Längsachse geführt sind.

Bei der Betätigungsvorrichtung kann es sich um eine mechanische Betätigungsvorrichtung, etwa eine Betätigungsstange, ein Betätigungsseil oder einen Betätigungsdraht oder eine Kombination aus den vorgenannten handeln. Die Betätigungsvorrichtung kann aber auch eine hydraulisch betätigbare Vorrichtung sein, die einen Betätigungskolben aufweist, der wiederum längsaxial zum ersten Schaftabschnitt verfahren werden kann.

Die operative Kopplung des Übertragungsarms mit dem Schieber kann im einfachsten Fall realisiert sein, indem das distale Ende des Übertragungs-arms bei einer Verschiebung in distaler Richtung entlang des Langlochs der Zapfen-Langloch-Verbindung den Schieber kontaktiert und dadurch die Bewegung auf den Schieber übertragen wird.

Retraktionsstrukturen an sich sind bekannt, sie bestehen aus mehreren Gliedern, üblicherweise mehr als fünf, die untereinander über Gelenke mit zumindest einem Bewegungsfreiheitsgrad verbunden sind.

Anders als bei Retraktoren gemäß dem Stand der Technik wird die Kopplungsvorrichtung beim erfindungsgemäßen Retraktor durch aktives Tun des Bedieners gesperrt und freigegeben, wobei zum Koppeln des Kopplungsendes der Retraktionsstruktur mit der Kopplungsvorrichtung keine Sperrkräfte, wie etwa eine Federkraft, überwunden werden muss; wenn die Kopplungsvorrichtung in ihren Freigabezustand versetzt ist, kann das Kopplungsende der Retraktionsstruktur ohne größere Kraftaufwendung mit ihr gekoppelt werden. Hierdurch wird erreicht, dass beim Koppeln oder Entkoppeln von der Kopplungsvorrichtung keine Gefahr, abzurutschen, mehr besteht; auch unter beengten räumlichen Verhältnissen in einem Operationsgebiet kann das Kopplungsende der Retraktionsstruktur daher sicher an den zweiten Schaftabschnitt gekoppelt werden, um den Ring zu bilden.

In einer weiteren Ausführungsform des erfindungsgemäßen Retraktors kann der Schieber eine Durchtrittsöffnung für das Kopplungsende der Retraktionsstruktur haben. Die Durchtrittsöffnung des Schiebers liegt in einem Freigabezustand der Kopplungsvorrichtung über einer Einstecköffnung, die in einer Wandung des zweiten Schaftabschnitts für das Kopplungsende der Retraktionsstruktur vorgesehen ist. Von der Durchtrittsöffnung erstreckt sich in distaler Richtung ein zweites Langloch, das in einem Sperrzustand der Kopplungsvorrichtung die Einstecköffnung in der Wandung des zweiten Schaftabschnitts zumindest teilweise überlappt. Die Abmessungen des aufzunehmenden Kopplungsendes der Retraktionsstruktur korrespondieren mit den Abmessungen des Langlochs.

Durch die längsaxiale Verschiebung des Schiebers wird also im Freigabezustand der Kopplungsvorrichtung ein "Durchtritt" für das Kopplungsende der Retraktionsstruktur geschaffen, während im Sperrzustand der Kopplungsvorrichtung das Kopplungsende durch die Zusammenwirkung der Einstecköffnung und des zweiten Langlochs des Schiebers formschlüssig gehalten wird. Insbesondere muss die in Bezug zum zweiten Schaftabschnitt radiale Position des Kopplungsendes der Retraktionsstruktur, die Breite des Langlochs in Bezug zu einem Durchmesser des Kopplungsendes und die Dicke des Schiebers auf eine Dicke des Kopplungsendes abgestimmt werden. Die Durchtrittsöffnung und das daran angrenzende zweite Langloch sind nicht getrennt voneinander sondern verbunden, so dass sich eine "schließzylinderförmige" Öffnung ergibt. In Zusammenwirkung dieser Öffnung, die gegenüber der Einstecköffnung verschiebbar ist, mit der Einstecköffnung ergibt sich die Lösbarkeit der Kopplungsvorrichtung.

In einer noch weiteren Ausführungsform kann das Kopplungsende der Retraktionsstruktur einen Sicherungspin umfassen, der bevorzugt eine Einschnürung aufweisen kann. Die Abmessungen der Einschnürung korrespondieren mit dem Langloch des Schiebers. Vorteilhaft kann der Sicherungspin auch einen pilzförmigen Kopf haben; ein kugelförmiger Kopf kann auch einsetzt werden, der aber weniger gut ist als ein Pilzkopf, da dieser in dem "Schloss" evtl. mehr Spiel hat.

Ein Sicherungspin mit kugelförmigem Kopf kann viel einfacher als ein Sicherungspin mit planem Kopf mit der Kopplungsvorrichtung in Eingriff gebracht werden, da er quasi seinen Weg in die Kopplungsvorrichtung selbsttätig findet. Auch erlaubt ein Sicherungspin mit kugelförmigem Kopf, ähnlich wie bei einem Kugelgelenk, eine Winkelbeweglichkeit in bestimmten Grenzen, was insbesondere zur Vermeidung von Verletzungen vom gehaltenem oder umliegenden Gewebe von Vorteil ist.

Gemäß einer weiteren Ausführungsform kann das Langloch der Zapfen-Langloch-Verbindung in einem distalen Endabschnitt des Übertragungsarms vorliegen. Dieses Langloch kann in Eingriff mit einem Zapfen stehen, der seinerseits verschiebefest in einem proximalen Endabschnitt des zweiten Schaftabschnitts angeordnet ist.

In fertigungstechnischer Hinsicht ist es deutlich einfacher, das Langloch der Zapfen-Langloch-Verbindung in dem Übertragungsarm zu fertigen, als in dem zweiten Schaftabschnitt. Funktionell ist es jedoch auch möglich, dass das Langloch in dem zweiten Schaftabschnitt vorliegt und der Zapfen fest mit dem Übertragungsarm verbunden ist.

Fertigungstechnisch wäre es auch möglich, das Langloch im zweiten Schaft-abschnitt zu fertigen, wobei das Langloch nicht zwingend innenliegend auszuführen ist. Hier wäre eine nicht durchgehende Kontur denkbar (quasi wie eine Sacklochbohrung) oder auch durchgehend im Sinne einer Durchgangsbohrung. Die Verbindung könnte dann mit einem Stift realisiert werden, im Übertragungsarm müsste hierfür eine entsprechende Bohrung ausgeführt werden. Aus fertigungstechnischer Sicht ist die Bearbeitung auf einer planen Oberfläche wie die des Übertragungsarmes einfacher als auf einer gewölbten Oberfläche wie die eines Zylinders.

Von der Funktion her wäre es jedoch denkbar, dass Bohrung und Langloch äquivalent getauscht werden.

Des Weiteren kann der Schieber an einem proximalen Ende eine Rinne aufweisen, die sich normal zu einer Schwenkebene der Schaftabschnitte erstreckt. In der Rinne ist das distale Ende des Übertragungsarms aufgenommen, wobei eine Außenkontur des Querschnitts des distalen Endes des Übertragungsarms mit einer Kontur des Querschnitts der Rinne korrespondiert.

"Querschnitt" ist hier in Bezug auf die Ausrichtung der Rinne zu verstehen und nicht in Bezug auf die Längsachse des Schaftes bzw. der Schaftabschnitte. Durch Zusammenwirkung der Rinne mit dem distalen Ende des Übertragungsarms kann eine formschlüssige Kopplung des Übertragungsarms mit dem Schieber erreicht werden. Über diese formschlüssige Kopplung kann der Schieber sowohl nach distal als auch nach proximal verfahren werden, indem der Übertragungsarm mittelbar durch die Betätigungsvorrichtung verfahren wird. Es kann sowohl die Kopplungsvorrichtung durch distal Bewegen des Schiebers ausgelöst werden als auch der Schieber zurück in seine Sperrposition gefahren werden. Es ist zur Rückholung des Schiebers nicht unbedingt eine Rückholvorrichtung, wie etwa eine Feder, nötig; der Schieber ist mit dem Übertragungsarm zwangsgekoppelt. Ferner wird die Bewegung des Übertragungsarms in einer nach distal weisenden Richtung durch die Zwangskopplung des distalen Endes mit der Rinne des Schiebers gesperrt, wenn die beiden Schaftabschnitte unter einem Winkel zueinander verschwenkt sind; sie ist nur freigegeben, wenn die Schaftabschnitte gestreckt sind. Hierdurch wird dem unbeabsichtigten Öffnen der Kopplungsvorrichtung durch externe Krafteinwirkung vorgebeugt.

Gemäß einer noch weiteren Ausführungsform kann die Rinne einen kreisförmigen Querschnitt haben, wobei der Mittelpunkt des kreisförmigen Querschnitts bevorzugt in einer Achse mit dem Zapfen der Zapfen-Langloch-Verbindung liegt. Da der Mittelpunkt der Rinne und die Achse des Zapfens gleich sind, bleibt die Längsachsenposition des Schiebers bei einer Rotation des zweiten Schaftabschnittes unverändert zu diesem. Das "Schloss", wie oben dargelegt, bleibt somit in abgewinkelter Stellung immer geschlossen. Wären die Drehzentren nicht identisch, würde sich bei einer Abwinklung des zweiten Schaftabschnitts der Schieber durch die Zwangsführung bewegen und hier das Schloss öffnen oder auch weiter schließen, was zu Verschleiß am Instrument oder im schlimmsten Fall zum Freigeben des Schlosses führen kann.

Darüber hinaus können der zweite Schaftabschnitt und der Übertragungsarm über eine Kulissenführung zwangsgekoppelt sein. Insbesondere kann der Übertragungsarm benachbart zu dem Langloch eine Führungsnut aufweisen, die sich entlang eines proximalen Nutabschnitts parallel zum Langloch erstreckt und entlang eines distalen Nutabschnitts parallel zur Querschnittskontur der Rinne. In der Führungsnut ist ein Führungsstift verschiebbar geführt, der verschiebefest an dem zweiten Schaftabschnitt angeordnet ist. Auch hier ist ein äquivalenter Austausch der miteinander operierenden Komponenten möglich: Die Nut kann am Schieber statt am Übertragungsarm, der den Schieber führt, vorliegen.

Die Richtungsangaben der Führungsnut sind jeweils auf den gestreckten Zustand der Schaftabschnitte bezogen. Durch die zusätzliche Zwangskopplung kann ein Spiel der beiden Schaftabschnitte zueinander, das ansonsten insbesondere bei kleinen Schwenkwinkeln auftritt, deutlich reduziert werden. Insbesondere lässt sich eine spielarme Kopplung der beiden Schaftabschnitte selbst bei vergleichsweise hohen Fertigungstoleranzen erreichen. Hat der Retraktor die zusätzliche Zwangsführung nicht, so tritt bei Einwirkung einer Radialkraft auf den zweiten Schaftabschnitt in Schwenkrichtung Spiel auf, das dem Spiel des Zapfens im Langloch der Zapfen-Langloch-Verbindung entspricht.

Des Weiteren kann, um ein ansprechendes Gestaltungsbild zu erreichen, die Führungsnut der Kulissenführung an zumindest ihrem distalen Ende geschlossen sein, wodurch zusätzlich eine Winkelbegrenzung erreicht werden kann, die an sich über das Gelenk erfolgt. Die Führungsnut kann sich etwa so weit nach distal erstrecken, dass eine Winkelbegrenzung erst bei einem Schwenkwinkel von 90° oder aber auch einem geringeren Winkel erfolgt.

Des Weiteren kann zwischen dem zweiten Schaftabschnitt und dem Schieber eine Rückstellvorrichtung für den Schieber angeordnet sein, bei der es sich insbesondere um eine Feder handeln kann. Die Rückstellvorrichtung ist dazu vorgesehen, insbesondere in einer Ausführungsform ohne formschlüssige Kopplung des Übertragungsarms mit dem Schieber, den Schieber in längsaxialer Richtung in eine Position zurückzuführen, die dem Sperrzustand der Kopplungsvorrichtung entspricht.

Darüber hinaus kann der zweite Schaftabschnitt über ein Gelenk an den ersten Schaftabschnitt angelenkt sein, wobei das Gelenk bevorzugt einen Körper aufweist, der mit einem der Schaftabschnitte verbunden ist. Der Körper des Gelenks kann benachbart zu einer Drehachse des Gelenks eine Führungsgasse aufweisen, die entlang eines Umfangabschnitts umläuft. In der Führungsgasse ist ein Stift des jeweils anderen Schaftabschnitts geführt.

Bei der Führungsgasse handelt es sich insbesondere um eine beidseitig geschlossene Führungsgasse, die zur zusätzlichen Winkelbegrenzung in beiden Endpositionen vorgesehen ist. In Umfangsrichtung bedeutet hierin, dass die Führungsgasse in konstantem Abstand zu der Drehachse des Gelenks verläuft, also kreisbogenförmig.

Der Körper des Gelenks kann ferner einen zylindrischen Aufnahmeabschnitt aufweisen, der bevorzugt in einer Aufnahmebohrung, etwa einer Sacklochbohrung, eines Schaftabschnitts aufgenommen ist. Die Aufnahmebohrung kann sich von einer zu dem jeweils anderen Schaftabschnitt weisenden Stirnfläche des jeweiligen Schaftabschnitts erstrecken. Es kann jedoch auch vorgesehen sein, dass das Gelenk ein "Doppelgelenk" ist, das über zwei Gelenkkörper verfügt, die jeweils an einem anderen Schaftabschnitt befestigt sind, und in denen jeweils ein Stift geführt ist. Indem der Gelenkkörper als separates Bauteil ausgeführt wird kann der gesamte Retraktor günstiger hergestellt werden, da die Fertigungstoleranzen bei der Herstellung des ersten und des zweiten Schaftabschnitts vergleichsweise weit gewählt werden können, da Wirkflächen nicht bearbeitet werden müssen bzw. Wirkflächen in das separate Bauteil ausgelagert sind.

Schließlich kann mit einem proximalen Ende des ersten Schaftabschnitts eine Handhabe verbunden sein, die ein oder mehrere Betätigungselement(e) aufweist. Zumindest ein Betätigungselement ist operativ mit der Betätigungsvorrichtung gekoppelt und dazu ausgebildet, die Betätigungsvorrichtung in einem vorbestimmten Verschiebewegbereich zu bewegen.

Bei dem Betätigungselement kann es sich etwa um ein Drehrad, einen Hebel, einen Schieber, einen Pinzettengriff und/oder einen Pistolenabzug handeln. Betätigungselemente sind bekannt und werden in verschiedenen Bauformen in endoskopischen und/oder laparoskopischen Instrumenten eingesetzt. Der erste Schaftabschnitt kann zur Erreichung eines Operationsgebiets innerhalb eines menschlichen oder tierischen Körpers eine Länge von etwa 10 cm bis über 60 cm aufweisen. Der vorbestimmte Verschiebewegbereich schließt hierbei den Verschiebeweg der Betätigungsvorrichtung in distale Richtung mit ein, der nötig ist, um die Kopplungsvorrichtung in den Freigabezustand zu überführen. Ein Bediener des Retraktors muss dabei darauf achten, das Betätigungselement nicht zu weit in distale Richtung zu bewegen, um die Freigabe der Kopplungsvorrichtung nicht versehentlich auszulösen.

Um dies zu verhindern kann ein zusätzlicher distaler Verschiebewegabschnitt des Betätigungselements der Handhabe freigegeben werden, wobei der zusätzliche Verschiebewegabschnitt wenigstens so lang ist wie eine Strecke des Schiebers zwischen seinem Freigabezustand und seinem Sperrzustand. Hierdurch kann erreicht werden, dass der Bediener die Freigabe der Kopplungsvorrichtung nicht versehentlich hervorrufen kann, sondern erst durch eine Aktion an der Handhabe den zur Auslösung vorgesehenen zusätzlichen Verschiebewegabschnitt freigeben muss. "Verschiebewegabschnitt" ist hierbei in Bezug auf die längsaxiale Bewegung der Betätigungsvorrichtung zu verstehen und nicht auf eine Bewegung des Betätigungselements, da zwischen einer Bewegung bzw. Rotation des Betätigungselements und der translatorischen Bewegung der Betätigungsvorrichtung stets ein bestimmtes Übersetzungsverhältnis vorgesehen sein wird.

Das erfindungsgemäße Bedienverfahren für den Retraktor weist in einer ersten Ausführungsform die folgenden Schritte auf:
a) in gestreckte Lage Bringen des ersten Schaftabschnitts und des zweiten Schaftabschnitts,
b) Verschieben der Betätigungsvorrichtung in distaler Richtung bis zu einer vorbestimmten Endposition, dadurch entlang des Langlochs der Zapfen-Langloch-Verbindung Verschieben des Übertragungsarms und Mitbewegen des Schiebers, dadurch Erreichen des Freigabezustands der Kopplungsvorrichtung,
c) Heranführen des Kopplungsendes der Retraktionsstruktur an die Kopplungsvorrichtung des zweiten Schaftabschnitts und Verbinden mit der Kopplungsvorrichtung,
d) Verschieben der Betätigungsvorrichtung in proximaler Richtung, dabei mit Verschieben des Schiebers und Überführen der Kopplungsvorrichtung in den Sperrzustand und Sperren des Kopplungsendes der Retraktionsstruktur in der Kopplungsvorrichtung.

Zum Lösen wird statt des Heranführens des Kopplungsendes der Retraktionsstruktur Herausziehen ausgeführt.

Im Weiteren kann nach Schritt d) Schritt e) ausgeführt werden: Weiter verschieben der Betätigungsvorrichtung in proximaler Richtung, dabei Mitverschieben des Übertragungsarms in proximaler Richtung und Kontakt Herstellen einer distalen Wandung des Langlochs des Übertragungsarms mit dem Zapfen und Übertragen der Bewegung der Betätigungsvorrichtung auf den zweiten Schaftabschnitt; hierdurch Erreichen einer Abwinkelbewegung des zweiten Schaftabschnitts.

Anders als bei bekannten Retraktoren, bei denen das Kopplungsende der Retraktionsstruktur über ein Federdruckstück mit dem zweiten Schaftabschnitt gekoppelt wird, ist das Einführen des Kopplungsendes mit den erfindungsgemäßen Verfahren unter Verwendung des erfindungsgemäßen Retraktors schneller und einfacher möglich, da keine Sperrkraft überwunden werden muss. Der Operateur läuft beim Koppeln weniger Gefahr, mit einer Fasszange o. ä. von der Retraktionsstruktur abzurutschen, wodurch die Ankopplung der Retraktionsstruktur schneller und schon nach weniger Fehlversuchen erfolgen kann, wodurch das gesamte Operationsgeschehen effizienter wird.

In einer weiteren Ausführungsform kann im Schritt c) die Betätigungsvorrichtung in distaler Richtung bis die Durchtrittsöffnung des Schiebers und die Einstecköffnung des zweiten Schaftabschnitts übereinander liegen verschoben werden. Zusätzlich wird im Schritt d) der Sicherungspin der Retraktionsstruktur in die Einstecköffnung des zweiten Schaftabschnitts durch die Durchtrittsöffnung des Schiebers gesteckt und im Schritt e) die Betätigungsvorrichtung in proximaler Richtung verschoben bis die Einstecköffnung des zweiten Schaftabschnitts das Langloch des Schiebers zumindest teilweise überlappt.

Ferner kann die Betätigungsvorrichtung mittels des Betätigungselements der Handhabe verschoben werden. Bevorzugt wird vor dem Schritt b) der Schritt a') Freigeben des zusätzlichen Verschiebewegabschnitts des Betätigungselements der Handhabe ausgeführt.

Diese und weitere Vorteile werden durch die nachfolgende Beschreibung unter Bezug auf die begleitenden Figuren dargelegt. Der Bezug auf die Figuren in der Beschreibung dient der Unterstützung der Beschreibung und dem erleichterten Verständnis des Gegenstands. Gegenstände oder Teile von Gegenständen, die im Wesentlichen gleich oder ähnlich sind, können mit denselben Bezugszeichen versehen sein. Die Figuren sind lediglich schematische Darstellungen von Ausführungsbeispielen der Erfindung. Es zeigen:
- **Fig. 1**: eine Draufsicht eines distalen Teils des Retraktors,
- **Fig. 2**: eine Teilschnittansicht der Schaftabschnitte in gestrecktem Zustand,
- **Fig. 3**: eine Teilschnittansicht der Schaftabschnitte in abgewinkeltem Zustand,
- **Fig. 4**: eine Seitenansicht eines Teils der Schaftabschnitte mit transparentem zweiten Schaftabschnitt und dem Schieber im Sperrzustand,
- **Fig. 5**: eine Seitenansicht eines Teils der Schaftabschnitte mit transparentem zweiten Schaftabschnitt und dem Schieber im Freigabezustand,
- **Fig. 6**: eine Seitenansicht eines Teils der Schaftabschnitte in abgewinkelten Zustand mit transparentem zweiten Schaftabschnitt,
- **Fig. 7**: einen Sicherungspin in perspektivischer Ansicht.

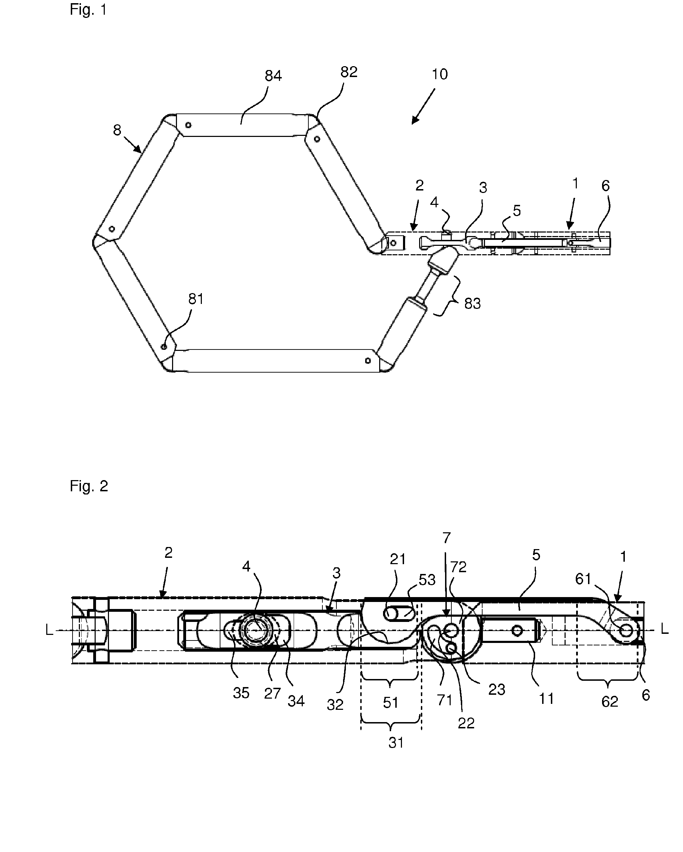

**Fig. 1** zeigt den erfindungsgemäßen Retraktor 10, der im Wesentlichen aus der Retraktionsstruktur 8 sowie dem ersten Schaftabschnitt 1 und zweiten Schaftabschnitt 2 besteht. Die Retraktionsstruktur 8 ist dazu vorgesehen, um unter oder um ein Organ, etwa unter die Leber, gelegt zu werden, um dieses aus einem Operationsgebiet heraus zu halten, wobei die Retraktionsstrur 8 in dem meisten Fällen unter einem Organ positioniert wird, um ein "blindes" Hantieren hinter dem Organ zu vermeiden. Die Retraktionsstruktur 8 hat mehrere Ringglieder 84, die über Gelenke um die Gelenkachsen jeweils schwenkbar miteinander verbunden sind. Der sichtbare Fixierstift 81 ist für die Fixierung der Gelenkkugel verantwortlich während die Gelenkachse verdeckt ist. Die Gelenke haben jeweils einen atraumatisch gerundeten Gelenkkörper 82, damit sich keine Gewebebestandteile darin verklemmen können. Die Retraktionsstruktur 8 ist an ihrem freien Ende mit dem distalen Ende des zweiten Schaftabschnitts 2 verbunden und mit ihrem anderen Ende im Wesentlichen quer zum zweiten Schaftabschnitt 2 ebenfalls mit diesem verbunden. Die Verbindung des freien Endes der Retraktionsstruktur 8 mit dem zweiten Schaftabschnitt 2 ist lösbar und wird bei einer Operation erst im Operationsfeld geschlossen, um die Größe des Zugangs / der Inzision zum Operationsfeld möglichst klein zu halten.

Das freie Ende hat ferner einen Fassbereich 83 für zusätzliches Werkzeug für Montage oder Demontage der Retraktionsstruktur 8, der so gestaltet ist, dass er bequem und abrutschsicher gegriffen werden kann.

Die Retraktionsstruktur 8 umfasst an ihrem "freien Ende" bzw. Kopplungs-nde einen Sicherungspin 4, der formschlüssig in Eingriff mit einer Kopplungsvorrichtung des zweiten Schaftabschnitts 2 steht, wobei die Kopplungsvorrichtung durch einen Schieber 3 freigegeben werden kann; hierzu wird der Schieber 3 mittels der Betätigungsvorrichtung 6 und des Übertragungsarms 5 in gestreckter Stellung in distaler Richtung verschoben, wodurch der formschlüssige Eingriff des Sicherungspins 4 in der Kopplungsvorrichtung aufgehoben wird. Beim Herausnehmen des Sicherungspins 4 aus der Kopplungsvorrichtung muss vorteilhaft keine Sperrkraft, wie sie etwa bei bekannten Retraktoren durch ein Federdruckstück erzeugt wird, überwunden werden, vielmehr kann der Sicherungspin 4 frei und ohne Kraftaufwand entkoppelt werden.

Der erste Schaftabschnitt 1 und der zweite Schaftabschnitt 2 sind schwenkbar verbunden, wobei die Schwenkebene senkrecht zur Bildebene der **Fig. 1** steht, so dass der zweite Schaftabschnitt 2 aus der Bildebene heraus geschwenkt werden kann. Hierzu ist im ersten Schaftabschnitt 1 eine Betätigungsstange 6 längsaxial verschiebbar geführt, deren distales Ende an einen Übertragungsarm 5 (siehe **Fig. 2**) angelenkt ist, der wiederum zur Erzeugung der Schwenkbewegung an den zweiten Schaftabschnitt 2 angelenkt ist.

Der erfindungsgemäße Retraktor 10 kann mittels einer herkömmlichen Handhabe, wie sie in Form von Pinzetten-, Pistolen-, Ring- und Scherenhandgriffen bekannt ist, betätigt werden, wobei die Handhabe an einem proximalen Ende des ersten Schaftabschnitts 1, das in einem Bereich rechts außerhalb des in der Figur dargestellten Bereichs liegt, mit dem ersten Schaftabschnitt 1 gekoppelt ist und mit dem die Betätigungsstange 6 betätigt werden kann. Geeigneterweise kommt eine Handhabe zum Einsatz, die das Erreichen einer proximalen "Überoffenstellung" erlaubt, wodurch das "Schloss", das durch den Schieber 3 und die Einstecköffnung 27 des zweiten Schaftabschnitts 2 gebildet wird, betätigt werden kann.

In **Fig. 2** ist die mechanische Umsetzung zur Freigabe der Kopplungsvorrichtung und zur Verschwenkung des zweiten Schaftabschnitts 1 in einer Teilansicht besser zu erkennen, wobei der erste 1 und der zweite Schaftabschnitt 2 entlang der Längsachse geschnitten dargestellt sind. Der zweite Schaftabschnitt 2 ist über ein Gelenk 7, dessen Gelenkkörper 72 in einem Sackloch 11 des ersten Schaftabschnitts 1 aufgenommen ist, um den Zapfen 22 drehbar gelagert.

An die Betätigungsstange 6 ist in ihrem distalen Endabschnitt 62 mittels eines Verbindungsstifts 61 der Übertragungsarm 5 angelenkt, der sich bis in einem proximalen Endabschnitt des zweiten Schaftabschnitts 2 erstreckt, wo er exzentrisch zur Längsachse L und exzentrisch zur Drehachse der beiden Schaftabschnitte 1,2 an den zweiten Schaftabschnitt 2 angelekt ist. Eine Zugbewegung der Betätigungsstange 6 bewirkt also eine Drehbewegung des zweiten Schaftabschnitts 2 um den Zapfen 22. Die Anlenkung des zweiten Schaftabschnitts 2 an den ersten Schaftabschnitt 1 ist mittels eines Gelenks 7, das einen Gelenkkörper 72 aufweist, realisiert. Zur Begrenzung des Verschwenkbereichs des zweiten Schaftabschnitts 2 liegt in dem, Gelenkkörper 72 eine Führungsgasse 71 vor, die auf einem Kreisbogen um den Zapfen 22 umlaufend ausgebildet ist und in der der Drehwinkelbegrenzungsstift 23 geführt ist.

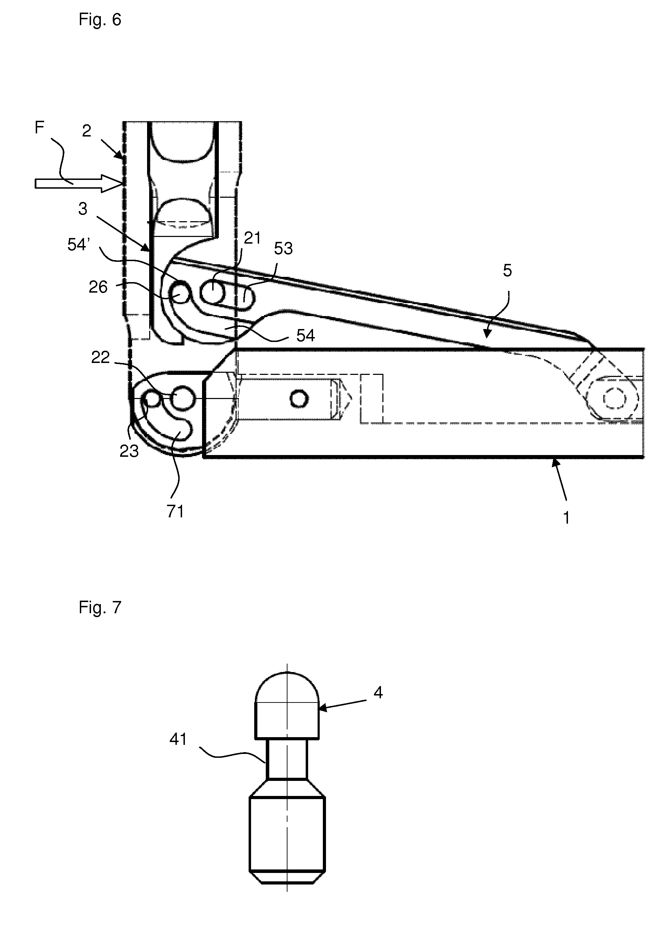

In **Fig. 2** ist nur der Sicherungspin 4 der Retraktionsstruktur 8 gezeigt, während die Ringglieder 84 selbst ausgeblendet sind. Die Kopplungsvorrichtung für den Sicherungspin 4 wird durch die Zusammenwirkung des Schiebers 3 mit der Wandung des zweiten Schaftabschnitts 2 gebildet. Der Schieber 3 hat eine Durchtrittsöffnung 34 für den Sicherungspin 4, an die sich distal ein zweites Langloch 35 anschließt, dessen Breite kleiner ist als der Durchmesser der Durchtrittsöffnung 34. Der Sicherungspin 4 hat insbesondere eine umlaufende Einschnürung (siehe **Fig. 7**) deren Durchmesser, Höhe und Position auf das Langloch 35 des Schiebers 3 abgestimmt sind. Der zweite Schaftabschnitt 2 weist eine Einstecköffnung 27 für den Sicherungspin 4 auf, die in gegenüberliegenden Abschnitten der Wandung des zweiten Schaftabschnitts 2 vorliegt und durch die der Sicherungspin 4 gesteckt werden kann sobald der Schieber nach distal verfahren ist und die Durchtrittsöffnung 34 über der Einstecköffnung 27 liegt. Zum Sperren wird der Schieber 3 in proximaler Richtung verfahren, bis das Langloch 35 die Einstecköffnung 27 überlappt und in Eingriff mit der Einschnürung (siehe **Fig. 7**) des Sicherungspins 4 steht. Die Kopplungsvorrichtung kann jedoch nur in gestrecktem Zustand der Schaftabschnitte 1,2 betätigt werden, während bei einer starken Abwinkelung aufgrund der Kraftrichtung der Betätigungsstange 6 keine Betätigung möglich ist.

Zur Kraftübertragung hat der Schieber 3 an seinem proximalen Ende 31 eine Rinne 32, die einen kreisförmigen Querschnitt aufweist, in der das distale Ende 51 des Übertragungsarms 5 aufgenommen ist. Die Querschnitte des distalen Endes des Übertragungsarms 5 und der Rinne 32 des Schiebers 3 korrespondieren sowohl in ihrer Form als auch in ihrer Abmessungen, wodurch eine spielarme Zwangskopplung erreicht wird und sowohl Zug- als auch Druckkräfte von dem Übertragungsarm 5 auf den Schieber 3 übertragen werden können. Die Richtung und die Strecke entlang der der Übertragungsarm verfahrbar ist wird durch das Langloch 53, das in dem distalen Endabschnitt 51 des Übertragungsarms 5 vorliegt, vorgegeben.

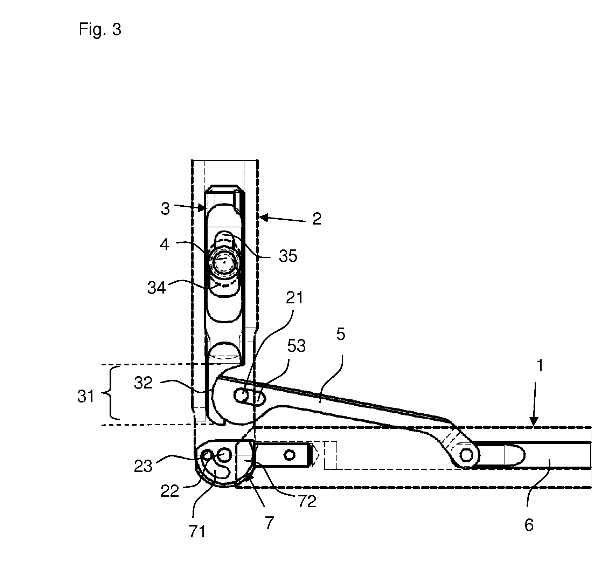

In **Fig. 3** sind die beiden Schaftabschnitte 1,2 in einem abgewinkelten Zustand dargestellt. Der zweite Schaftabschnitt 2 kann von dieser Lage ausgehend nicht weiter ausgeschwenkt werden, da der Drehwinkelbegrenzungsstift 23, der in der Führungsgasse 71 des Gelenkkörpers 72 geführt ist, an dem Ende der Führungsgasse 71 anschlägt.

Bei Einwirkung einer externen Radialkraft auf den zweiten Schaftabschnitt 2 tritt jedoch Spiel in der Mechanik auf, insbesondere um die Drehachse 22, dessen Größe maximal der Länge des Langlochs 53 entspricht. Das Spiel wird vor allem von Fertigungstoleranzen in der Führung des verrundeten distalen Endes des Übertragungsarms 5 in der Rinne 32 und einem Radialspiel des Schiebers 3 in seiner längsaxialen Führung im zweiten Schaftabschnitt 2 hervorgerufen. Neben den genannten toleranzbehafteten Wirkverbindungen können auch weitere Fertigungstoleranzen, etwa im Gelenk 22 oder in der Anlenkung des Übertragungsarms 4 an das Betätigungselement 6 zu vermehrtem Spiel um die Drehachse 22 führen.

Daneben tritt, insbesondere bei einer Ausführung der Schaftabschnitte 1,2 aus Kunststoff, elastische Biegung auf. Da die Führungsgasse 71 und der Stift 23 sehr nahe der Drehachse der beiden Schaftabschnitte 1,2 liegt, treten dort sehr große Sperrkräfte auf, was bei einer Belastung des zweiten Schaftabschnitts mit einer Radialkraft zu einer elastischen Durchbiegung des zweiten Schaftabschnitts führen kann, infolgedessen sich auch der Zapfen 21 im Langloch 53 bewegen wird. Bei einer Ausführung der Schaftab-schnitte 1,2 aus Stahl wird die elastische Durchbiegung jedoch unter den typischen Lasten gering ausfallen. Sollte Spiel auftreten, besteht trotzdem nicht die Gefahr, dass der Schieber 3 bewegt wird und die Kopplungsvorrichtung dadurch unbeabsichtigt in den Freigabezustand überführt wird.

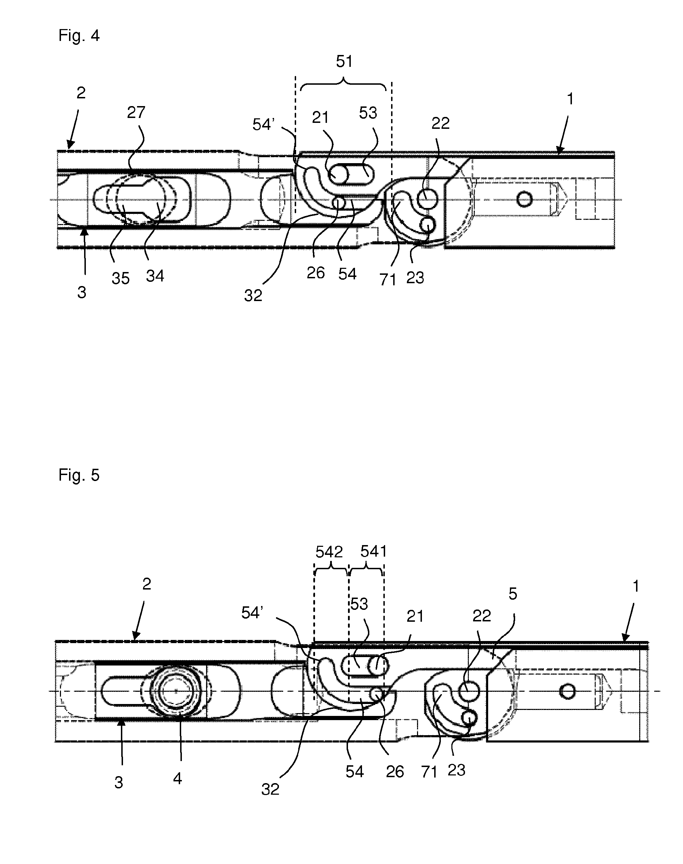

Um das Spiel in der Anlenkung der beiden Schaftabschnitte 1,2 zu reduzieren wird erfindungsgemäß die in den **Fig. 4** bis **Fig. 6** dargestellte Maßnahme vorgeschlagen.

Benachbart zu dem Langloch 53 liegt eine Führungsnut 54 im distalen Endabschnitt des Übertragungsarms 5 vor. Die Führungsnut 54 hat einen proximalen Nutabschnitt 541, in dem sie parallel zum Langloch 53 verläuft, und einen distalen Nutabschnitt 542, in dem sie der Querschnittskontur der Rinne 32 folgt. Der proximale Nutabschnitt 541 ermöglicht es dabei, dass der Schieber 3 weiterhin zur Freigabe der Kopplungsvorrichtung entlang des Langlochs 53 verschoben werden kann, während der distale Nutabschnitt 542 verhindern soll, dass bei externer Radialkrafteinwirkung auf den zweiten Schaftabschnitt 2 Spiel auftritt. Weitere Ausführungsformen, bei denen die Führungsnut 54 jedoch am Schieber 3 sitzt, sind möglich.

Die Kopplungsvorrichtung ist in **Fig. 4** im Sperrzustand dargestellt; der Schieber 3 ist nach proximal verfahren und der Zapfen 21 liegt an seinem distalen Anschlag an. Die Einstecköffnung 27 des zweiten Schaftabschnitts 2 wird durch das Langloch 35 im Schieber versperrt. **Fig. 5** hingegen zeigt den Freigabezustand der Kopplungsvorrichtung; der Schieber 3 ist ganz nach distal verschoben und der Zapfen 21 liegt an seinem proximalen Anschlag an. In diesem Zustand kann der Sicherungspin 4 in die Einstecköffnung 27 und durch die Durchtrittsöffnung des Schiebers 3 gesteckt werden, da die Durchtrittsöffnung 34 genau über der Einstecköffnung 27 liegt.

In **Fig. 6** ist eine abgewinkelte Stellung gezeigt. Das geschlossene Ende 54' der Führungsnut 54 stellt eine lediglich zusätzliche Winkelbegrenzung durch ihr geschlossenes Ende 54' bereit. Zudem tritt durch Einsatz der Kulissenführung, die durch die Führungsnut 54 und den Führungsstift 26 gebildet wird, ein geringes Spiel bei externer Radialkraftbelastung des zweiten Schaftabschnitts 2 auf. Bei einer Belastung in der mit dem Pfeil F dargestellten Richtung, würde sich ohne die Kulissenführung der zweite Schaftabschnitt 2 entsprechend eines Gesamtspiels, das sich aus dem genannten Komponenten zusammensetzt, verschwenken lassen. Die Kulissenführung verhindert dies, da der gebogene distale Nutabschnitt 542 (siehe **Fig. 5**) nahezu rechtwinklig zur Kraftrichtung F steht; die Verschwenkung des zweiten Schaftabschnitt 2 wird formschlüssig gesperrt bzw. auf einen Betrag reduziert, der dem Querspiel des Führungsstifts 26 in der Führungsnut 54 entspricht.

In **Fig. 7** ist der Sicherungspin 4 gezeigt, der dazu vorgesehen ist, mit seiner planen Stirnfläche an dem distalen Ende einer Retraktionsstruktur 8 (siehe **Fig. 1**) angeordnet zu werden. Er zeigt einen pilzförmigen Kopf, der für den Eingriff mit dem Langloch des Schiebers 3 (siehe **Fig. 2** bis **Fig. 6**) gestaltet ist.

**Bezugszeichenliste**

| | |
|---|---|
| 1 | Erster Schaftabschnitt |
| 10 | distaler Teil des Retraktors |
| 11 | Sackloch des ersten Schaftabschnitts |
| 2 | Zweiter Schaftabschnitt |
| 21 | Zapfen des zweiten Schaftabschnitt |
| 22 | Zapfen/Drehachse |
| 23 | Stift/Drehwinkelbegrenzungsstift |
| 26 | Führungsstift der Kulissenführung |
| 27 | Einstecköffnung des zweiten Schaftabschnitts |
| 3 | Schieber |
| 31 | Proximaler Endabschnitt des Schiebers |
| 32 | Rinne des Schiebers |
| 34 | Durchtrittsöffnung |
| 35 | Zweites Langloch, Langloch des Schiebers |
| 4 | Sicherungspin |
| 41 | Einschnürung |
| 5 | Übertragungsarm |
| 51 | Distaler Endabschnitt des Übertragungsarms |
| 53 | Langloch des Übertragungsarms |
| 54 | Führungsnut der Kulissenführung |
| 54' | Distales Ende der Führungsnut |
| 541 | Proximaler Nutabschnitt |
| 542 | Distaler Nutabschnitt |
| 6 | Betätigungsstange |
| 61 | Verbindungsstift |
| 62 | Distaler Endabschnitt der Betätigungsstange |
| 7 | Gelenk des orstfesten Schaftabschnitts |
| 71 | Führungsgasse des Gelenks |
| 72 | Gelenkkörper |
| 8 | Retraktionsstruktur |
| 81 | Fixierstift einer Gelenkkugel |
| 82 | Gelenkkörper |
| 83 | Fassbereich für zusätzliches Werkzeug für die Demontge/Montage |
| 84 | Glied der Retraktionsstruktur |

## Patentansprüche

1. Retraktor (10) für die endoskopische Chirurgie, umfassend
- einen ersten Schaftabschnitt (1), innerhalb dessen eine Betätigungsvorrichtung (6) verschiebbar geführt ist und der schwenkbar mit einem zweiten Schaftabschnitt (2) gekoppelt ist, und - einen Übertragungsarm (5), der einenends an ein distales Ende der Betätigungsvorrichtung (6) angelenkt ist und anderenends exzentrisch zu einer Drehachse (22) der Schaftabschnitte (1,2) an den zweiten Schaftabschnitt (2) angelenkt ist, und - eine Retraktionsstruktur (8), die einenends mit einem distalen Ende des zweiten Schaftabschnitts (2) verbunden ist und anderenends ein Kopplungsende aufweist, mit dem sie über eine Kopplungsvorrichtung mit dem zweiten Schaftabschnitt (2) lösbar koppelbar ist, **dadurch gekennzeichnet**, **dass-** die Kopplungsvorrichtung einen Schieber (3) aufweist, der innerhalb des zweiten Schaftabschnitts (2) verschiebbar geführt ist, und - der Übertragungsarm (5) mittels einer Zapfen-Langloch-Verbindung an den zweiten Schaftabschnitt (2) angelenkt ist, wobei sich das Langloch (53) der Zapfen-Langloch-Verbindung bei gestreckten Schaftabschnitten (1,2) parallel zu der Längsachse (L) erstreckt, - und wobei der Übertragungsarm (5) mit dem Schieber (3) zu dessen Längsverfahrung operativ gekoppelt ist, und wobei die Längsverfahrung des Schiebers (3) einen Freigabezustand der Kopplungsvorrichtung bereitstellt.

2. Retraktor (10) nach Anspruch 1, **dadurch gekennzeichnet, dass** der Schieber (3) eine Durchtrittsöffnung (34) für das Kopplungsende der Retraktionsstruktur (8) aufweist, die in dem Freigabezustand der Kopplungsvorrichtung über einer Einstecköffnung (27) in der eine Wandung des zweiten Schaftabschnitts (2) für das Kopplungsende der Retraktionsstruktur (8) liegt, und wobei - sich von der Durchtrittsöffnung (34) in distaler Richtung ein zweites Langloch (35) erstreckt, das in einem Sperrzustand der Kopplungsvorrichtung die Einstecköffnung (27) zumindest teilweise überlappt, wobei die Abmessungen des zweiten Langlochs (35) mit den Abmessungen des aufzunehmenden Abschnitts des Kopplungsendes der Retraktionsstruktur (8) korrespondieren.

3. Retraktor (10) nach Anspruch 2, **dadurch gekennzeichnet, dass** das Kopplungsende der Retraktiosstruktur (8) einen Sicherungspin (4) umfasst, der bevorzugt eine Einschnürung (41) aufweist, deren Abmessungen mit dem Langloch (35) des Schiebers (3) korrespondieren, wobei der Sicherungspin (4) besonders bevorzugt einen pilzförmigen Kopf hat.

4. Retraktor (10) nach zumindest einem der Ansprüche 1 bis 3, **dadurch gekennzeichnet, dass -** das Langloch (53) der Zapfen-Langloch-Verbindung an einem distalen Endabschnitt (51) des Übertragungsarms (5) vorliegt und in Eingriff mit einem Zapfen (21) steht, der verschiebefest in einem proximalen Endabschnitt des zweiten Schaftabschnitts (2) angeordnet ist, oder dass - das Langloch (53) der Zapfen-Langloch-Verbindung in einem proximalen Endabschnitt des zweiten Schaftabschnitts (52) vorliegt und in Eingriff mit einem Zapfen (21) steht, der verschiebefest an einem distalen Endabschnitt (51) des Übertragungsarms (5) angeordnet ist.

5. Retraktor (10) nach zumindest einem der Ansprüche 1 bis 4, **dadurch gekennzeichnet, dass** der Schieber (3) an einem proximalen Ende (31) eine Rinne (32) aufweist, die sich normal zu einer Schwenkebene der Schaftabschnitte (1,2) erstreckt und in der das distale Ende des Übertragungsarms (5) aufgenommen ist, wobei eine Außenkontur des Querschnitts des distalen Endes des Übertragungsarms (5) mit einer Querschnittskontur der Rinne (32) korrespondiert.

6. Retraktor (10) nach Anspruch 5, **dadurch gekennzeichnet, dass** die Rinne (32) einen kreisförmigen Querschnitt hat, wobei bevorzugt ein Mittelpunkt des kreisförmigen Querschnitts der Rinne (32) in einer Achse des Zapfens (21) der Zapfen-Langloch-Verbindung liegt

7. Retraktor (10) nach Anspruch 5 oder 6, **dadurch gekennzeichnet, dass** der zweite Schaftabschnitt (2) und der Übertragungsarm (5) über eine Kulissenführung zwangsgekoppelt sind, wobei an einem Bauteil Übertragungsarm (5) oder zweiter Schaftabschnitt benachbart zu dem Langloch (53) zumindest eine Führungsnut (54) vorliegt, die sich entlang eines proximalen Nutabschnitts (541) parallel zu dem Langloch (53) erstreckt und entlang eines distalen (542) Nutabschnitts parallel zu der Querschnittskontur der Rinne (32) erstreckt, wobei in der Führungsnut (54) ein Führungsstift (26) verschiebbar geführt ist, der verschiebefest an dem entsprechend korrespondierenden zweiten Bauteil Übertragungsarm (5) oder zweiter Schaftabschnitt (2) angeordnet ist.

8. Retraktor (10) nach zumindest einem der Ansprüche 1 bis 7, **dadurch gekennzeichnet, dass** zwischen dem zweiten Schaftabschnitt (2) und dem Schieber (3) eine Rückstellvorrichtung für den Schieber (3) angeordnet ist.

9. Retraktor (10) nach zumindest einem der Ansprüche 1 bis 8, **dadurch gekennzeichnet, dass** der zweite Schaftabschnitt (2) über ein Gelenk (7) an den ersten Schaftabschnitt (1) angelenkt ist, wobei das Gelenk (7) bevorzugt einen Körper (72) aufweist, der mit einem der Schaftabschnitte (1,2) verbunden ist und benachbart zu einer Drehachse des Gelenks (7) eine Führungsgasse (71) entlang eines Umfangsabschnitts umlaufend aufweist, in der ein Stift (23) des jeweils anderen Schaftabschnitts (1,2) geführt ist.

10. Retraktor (10) nach Anspruch 9, **dadurch gekennzeichnet, dass** der Körper (72) des Gelenks (7) einen zylindrischen Aufnahmeabschnitt aufweist, der bevorzugt in einer Aufnahmebohrung, bevorzugt einem Sackloch (11), des ersten Schaftabschnitts (1) oder des zweiten Schaftabschnitts (2) aufgenommen ist, die sich von einer zu dem jeweils anderen Schaftabschnitt (2) weisenden Stirnfläche erstreckt.

11. Retraktor (10) nach zumindest einem der Ansprüche 1 bis 10, **dadurch gekennzeichnet, dass** mit einem proximalen Ende des ersten Schaftabschnitts (1) eine Handhabe verbunden ist, die zumindest ein Betätigungselement aufweist, das operativ mit der Betätigungsvorrichtung (6) gekoppelt und das dazu ausgebildet ist, die Betätigungsvorrichtung (6) in einem vorbestimmten Verschiebewegbereich zu bewegen.

12. Retraktor nach Anspruch 11, **dadurch gekennzeichnet, dass** ein zusätzlicher distaler Verschiebewegabschnitt des Betätigungselements der Handhabe freigebbar ist, der zumindest so lang ist wie eine Strecke des Schiebers (3) zwischen seinem Freigabezustand und seinem Sperrzustand.

## Claims

1. Retractor (10) for endoscopic surgery, comprising
- a first shank portion (1), within which an actuation device (6) is guided movably, and which is pivotably coupled to a second shank portion (2), and
- a transmission arm (5), which at one end is articulated on a distal end of the actuation device (6) and at the other end is articulated, eccentrically with respect to a rotation axis (22) of the shank portions (1, 2), on the second shank portion (2), and
- a retraction structure (8), which at one end is connected to a distal end of the second shank portion (2) and at the other end has a coupling end with which it can be releasably coupled to the second shank portion (2) via a coupling device,
**characterized in that**
- the coupling device has a slide (3), which is guided movably within the second shank portion (2), and
- the transmission arm (5) is articulated on the second shank portion (2) by means of a peg-and-slot connection, wherein the slot (53) of the peg-and-slot connection extends parallel to the longitudinal axis (L) when the shank portions (1, 2) are extended, and
- wherein the transmission arm (5) is operatively coupled to the slide (3) in order to move the latter longitudinally, and wherein the longitudinal travel of the slide (3) makes available a release state of the coupling device.

2. Retractor (10) according to Claim 1, **characterized in that** the slide (3) has a through-opening (34) for the coupling end of the retraction structure (8), which through-opening (34), in the release state of the coupling device, lies over an insertion opening (27) in the one wall of the second shank portion (2) for the coupling end of the retraction structure (8), and wherein a second slot (35) extends from the through-opening (34) in the distal direction and, in a locked state of the coupling device, at least partially overlaps the insertion opening (27), wherein the dimensions of the second slot (35) correspond to the dimensions of that portion of the coupling end of the retraction structure (8) that is to be received.

3. Retractor (10) according to Claim 2, **characterized in that** the coupling end of the retraction structure (8) comprises a securing pin (4), which preferably has a throat (41) whose dimensions correspond to the slot (35) of the slide (3), wherein the securing pin (4) particularly preferably has a mushroom-shaped head.

4. Retractor (10) according to at least one of Claims 1 to 3, **characterized in that** the slot (53) of the peg-and-slot connection is present on a distal end portion (51) of the transmission arm (5) and engages with a peg (21) which is arranged in a fixed position in a proximal end portion of the second shank portion (2), or **in that** the slot (53) of the peg-and-slot connection is present in a proximal end portion of the second shank portion (52) and engages with a peg (21) which is arranged in a fixed position on a distal end portion (51) of the transmission arm (5).

5. Retractor (10) according to at least one of Claims 1 to 4, **characterized in that** the slide (3) has, at a proximal end (31), a channel (32) which extends perpendicularly with respect to a pivot plane of the shank portions (1, 2) and in which the distal end of the transmission arm (5) is received, wherein an outer contour of the cross section of the distal end of the transmission arm (5) corresponds to a cross-sectional contour of the channel (32).

6. Retractor (10) according to Claim 5, **characterized in that** the channel (32) has a circular cross section, wherein a center point of the circular cross section of the channel (32) preferably lies in an axis of the peg (21) of the peg-and-slot connection.

7. Retractor (10) according to Claim 5 or 6, **characterized in that** the second shank portion (2) and the transmission arm (5) are positively coupled via a slotted guide, wherein at least one guide groove (54) is present on one component of transmission arm (5) and second shank portion adjacent to the slot (53), which guide groove (54) extends along a proximal groove portion (541) parallel to the slot (53) and extends along a distal groove portion (542) parallel to the cross-sectional contour of the channel (32), wherein a guide pin (26) is guided movably in the guide groove (54) and is arranged in a fixed position on the corresponding second component of transmission arm (5) and second shank portion (2).

8. Retractor (10) according to at least one of Claims 1 to 7, **characterized in that** a resetting device for the slide (3) is arranged between the second shank portion (2) and the slide (3).

9. Retractor (10) according to at least one of Claims 1 to 8, **characterized in that** the second shank portion (2) is articulated on the first shank portion (1) via a hinge (7), wherein the hinge (7) preferably has a body (72) which is connected to one of the shank portions (1, 2) and, adjacent to a rotation axis of the hinge (7), has a guide track (71) extending along a circumferential portion, in which a pin (23) of the respective other shank portion (1, 2) is guided.

10. Retractor (10) according to Claim 9, **characterized in that** the body (72) of the hinge (7) has a cylindrical receiving portion, which is preferably received in a receiving bore, preferably a blind hole (11), of the first shank portion (1) or of the second shank portion (2), which bore extends from an end face directed toward the respective other shank portion (2).

11. Retractor (10) according to at least one of Claims 1 to 10, **characterized in that** a handle is connected to a proximal end of the first shank portion (1) and has at least one actuation element which is operatively coupled to the actuation device (6) and which is designed to move the actuation device (6) in a predetermined range of travel.

12. Retractor according to Claim 11, **characterized in that** an additional distal range of travel of the actuation element of the handle can be freed, which is at least as long as a travel of the slide (3) between its release state and its locked state.

## Revendications

1. Rétracteur (10) pour la chirurgie endoscopique, comprenant:
- une première partie de tige (1), à l'intérieur de laquelle un dispositif d'actionnement (6) est guidé de façon coulissante et qui est couplée de façon pivotante à une deuxième partie de tige (2), et
- un bras de transmission (5), qui est articulé par une extrémité à une extrémité distale du dispositif d'actionnement (6) et qui est articulé par l'autre extrémité à la deuxième partie de tige (2) de façon excentrique par rapport à l'axe de rotation (22) des parties de tige (1, 2), et
- une structure de rétraction (8), qui est reliée par une extrémité à une extrémité distale de la deuxième partie de tige (2) et qui présente à l'autre extrémité une extrémité de couplage, avec laquelle elle peut être couplée de façon détachable à la deuxième partie de tige (2) par l'intermédiaire d'un dispositif de couplage,
**caractérisé en ce que**
- le dispositif de couplage présente un coulisseau (3), qui est guidé de façon coulissante à l'intérieur de la deuxième partie de tige (2), et
- le bras de transmission (5) est articulé à la deuxième partie de tige (2) par une liaison tourillon-trou oblong, dans laquelle le trou oblong (53) de la liaison tourillon-trou oblong s'étend parallèlement à l'axe longitudinal (L) lorsque les parties de tige (1, 2) sont étendues, et
- dans lequel le bras de transmission (5) est couplé activement au coulisseau (3) en vue de son déplacement longitudinal, et dans lequel le déplacement longitudinal du coulisseau (3) fournit un état de libération du dispositif de couplage.

2. Rétracteur (10) selon la revendication 1, **caractérisé en ce que** le coulisseau (3) présente une ouverture de passage (34) pour l'extrémité de couplage de la structure de rétraction (8), qui dans l'état de libération du dispositif de couplage se trouve au-dessus d'une ouverture d'introduction (27) dans l'une paroi de la deuxième partie de tige (2) pour l'extrémité de couplage de la structure de rétraction (8), et dans lequel un deuxième trou oblong (35) s'étend à partir de l'ouverture de passage (34) en direction distale, qui recouvre au moins en partie l'ouverture d'introduction (27) dans un état de blocage du dispositif de couplage, dans lequel les dimensions du deuxième trou oblong (35) correspondent aux dimensions de la partie à recevoir de l'extrémité de couplage de la structure de rétraction (8).

3. Rétracteur (10) selon la revendication 2, **caractérisé en ce que** l'extrémité de couplage de la structure de rétraction (8) comprend une broche de sécurité (4), qui présente de préférence un étranglement (41) dont les dimensions correspondent au trou oblong (35) du coulisseau (3), dans lequel la broche de sécurité (4) a en particulier de préférence une tête en forme de champignon.

4. Rétracteur (10) selon au moins une des revendications 1 à 3, **caractérisé en ce que** le trou oblong (53) de la liaison tourillon-trou oblong se trouve à une partie d'extrémité distale (51) du bras de transmission (5) et est en prise avec un tourillon (21), qui est disposé de façon solidaire en déplacement dans une partie d'extrémité proximale de la deuxième partie de tige (2), ou **en ce que** le trou oblong (53) de la liaison tourillon-trou oblong se trouve dans une partie d'extrémité proximale de la deuxième partie de tige (52) et est en prise avec un tourillon (21), qui est disposé de façon solidaire en déplacement sur une partie d'extrémité distale (51) du bras de transmission (5).

5. Rétracteur (10) selon au moins une des revendications 1 à 4, **caractérisé en ce que** le coulisseau (3) présente à une extrémité proximale (31) une rigole (32), qui s'étend normalement à un plan de pivotement des parties de tige (1, 2) et dans laquelle est logée l'extrémité distale du bras de transmission (5), dans lequel un contour extérieur de la section transversale de l'extrémité distale du bras de transmission (5) correspond à un contour de section transversale de la rigole (32).

6. Rétracteur (10) selon la revendication 5, **caractérisé en ce que** la rigole (32) a une section transversale circulaire, dans lequel un point central de la section transversale circulaire de la rigole (32) est situé dans un axe du tourillon (21) de la liaison tourillon-trou oblong.

7. Rétracteur (10) selon la revendication 5 ou 6, **caractérisé en ce que** la deuxième partie de tige (2) et le bras de transmission (5) sont en couplage forcé par un guide de coulisse, dans lequel il se trouve sur un composant, bras de transmission (5) ou deuxième partie de tige, à proximité du trou oblong (53), au moins une rainure de guidage (54), qui s'étend le long d'une partie de rainure proximale (541) parallèlement au trou oblong (53) et le long d'une partie de rainure distale (542) parallèlement au contour de section transversale de la rigole (32), dans lequel une tige de guidage (26) est guidée de façon coulissante dans la rainure de guidage (54) et est disposée de façon solidaire en déplacement sur le deuxième composant respectivement correspondant bras de transmission (5) ou deuxième partie de tige (2).

8. Rétracteur (10) selon au moins une des revendications 1 à 7, **caractérisé en ce qu'**un dispositif de rappel pour le coulisseau (3) est disposé entre la deuxième partie de tige (2) et le coulisseau (3).

9. Rétracteur (10) selon au moins une des revendications 1 à 8, **caractérisé en ce que** la deuxième partie de tige (2) est articulée à la première partie de tige (1) par une articulation (7), dans lequel l'articulation (7) présente de préférence un corps (72), qui est relié à une des parties de tige (1, 2) et qui présente, à proximité d'un axe de rotation de l'articulation (7), une voie de guidage (71) courant le long d'une partie de périphérie, dans laquelle un tenon (23) de l'autre partie de tige (1, 2) respective est guidé.

10. Rétracteur (10) selon la revendication 9, **caractérisé en ce que** le corps (72) de l'articulation (7) présente une partie de réception cylindrique, qui est logée de préférence dans un alésage de réception, de préférence un trou borgne (11), de la première partie de tige (1) ou de la deuxième partie de tige (2), qui s'étend à partir d'une face frontale tournée chaque fois vers l'autre partie de tige (2).

11. Rétracteur (10) selon au moins une des revendications 1 à 10, **caractérisé en ce qu'**une poignée est assemblée à une extrémité proximale de la première partie de tige (1), et présente au moins un élément d'actionnement, qui est couplé activement avec le dispositif d'actionnement (6) et qui est conçu pour déplacer le dispositif d'actionnement (6) dans une zone de déplacement prédéterminée.

12. Rétracteur selon la revendication 11, **caractérisé en ce qu'**une zone de déplacement distale supplémentaire de l'élément d'actionnement de la poignée peut être libérée, qui est au moins aussi longue qu'une course du coulisseau (3) entre son état de libération et son état de blocage.
